(19) Europäisches Patentamt
European Patent Office
Office européen des brevets

(11) Publication number: **0 202 589 B1**

(12) # EUROPEAN PATENT SPECIFICATION

(45) Date of publication of patent specification: **28.02.90**

(51) Int. Cl.[5]: **A 61 K 31/375,** C 07 D 307/62

(21) Application number: **86106521.7**

(22) Date of filing: **14.05.86**

(54) **Pharmaceutical compositions containing ascorbic acid derivatives.**

(30) Priority: **17.05.85 PCt/jp85/00272**
**18.06.85 PCt/jp85/00340**

(43) Date of publication of application:
**26.11.86 Bulletin 86/48**

(45) Publication of the grant of the patent:
**28.02.90 Bulletin 90/09**

(84) Designated Contracting States:
**AT BE CH DE FR GB IT LI LU NL SE**

(56) References cited:
**EP-A-0 074 411**
**DE-A-1 468 406**
**DE-A-1 518 988**
**DE-A-2 729 903**

**CHEMICAL ABSTRACTS, vol. 83, no. 21, 24th November 1975, page 622, abstract no. 179514j, Columbus, Ohio, US; & JP-A-75 30 864 (WAKAMOTO PHARMACEUTICAL CO. LTD) 27-03-1975**

**CHEMICAL ABSTRACTS, vol. 75, no. 21, 22nd November 1971, page 321, abstract no. 130075n, Columbus, Ohio, US; & JP-A-71 24 699 (SHIONOGI AND CO., LTD) 15-07-1971**

(73) Proprietor: **Takeda Chemical Industries, Ltd.**
**27, Doshomachi 2-chome Higashi-ku**
**Osaka-shi Osaka-fu (JP)**

(72) Inventor: **Terao, Shinji**
**26-3, Shinsenri-minamimachi 2-chome**
**Toyonaka-shi Osaka 565 (JP)**
Inventor: **Hirata, Minoru**
**26-13, Fushiodai 1-chome**
**Ikeda-shi Osaka 563 (JP)**

(74) Representative: **von Kreisler, Alek, Dipl.-Chem. et al**
**Deichmannhaus am Hauptbahnhof**
**D-5000 Köln 1 (DE)**

(56) References cited:
**CHEMICAL ABSTRACTS, vol. 73, no. 19, 9th November 1970, page 396, abstract no. 99173a, Columbus, Ohio, US; & JP-A-70 11 690 (SHIONOGI AND CO., LTD) 27-04-1970**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 202 589 B1**

(56) References cited:

JOURNAL OF AGRICULTURAL AND FOOD CHEMISTRY, vol. 32, no. 1, January 1984, pages 21-28, American Chemical Society, Washington, DE, US; P.W. LU et al.: "Synthesis of the 2-methyl ether of l-ascorbic acid: stability, vitamin activity, and carbon-13 nuclear magnetic resonance spectrum compared to those of the 1- and 3-methyl ethers"

CHEMICAL ABSTRACTS, vol. 69, no. 17, 21st October 1968, page 6239, abstract no. 66921g, Columbus, Ohio, US; & JP-A-67 26 633 (SHIONOGI AND CO., LTD) 16-12-1967

CHEMICAL ABSTRACTS, vol. 103, no. 4, 29th July 1985, page 292, abstract no. 27082k, Columbus, Ohio, US; & JP-A-60 23 307 (KANEBO LTD et al.) 05-02-1985

CHEMICAL ABSTRACTS, vol. 102, no. 19, 13th May 1985, page 528, abstract no. 165694s, Columbus, Ohio, US; G. TAO et al.: "Hypolipidemic and lipotropic effects of L-ascorbate 2-sulfate", & YINGYANG XUEBAO 1984, 6(3), 223-30

CHEMISTRY LETTERS, no. 5, May 1983, pages 631-632, Chemical Society of Japan, Tokyo, JP; E. NIKI et al.: "The role of vitamin C as an antioxidant"

JOURNAL OF ORGANIC CHEMISTRY, vol. 50, no. 2, 25th January 1985, pages 281-283, American Chemical Society, Easton, PA, US; W.A. PRYOR et al.: "Autoxidation of micelle-solubilized linoleic acid. Relative inhibitory efficiencies of ascorbate and ascorbyl palmitate"

## Description

This invention relates to pharmaceutical compositions containing ascorbic acid derivatives useful for prophylaxis and improvement of disorders in the circulatory system.

Diseases of heart, brain, kidney, etc., which are often observed in adults, are mainly caused by disturbances and destruction of cells or tissues due to ischemia as a basal pathologic state, to result in hemostasia leading to suspension of supplying energy source. The morbidity rate of, for example, ischemic heart diseases, ischemic cerebral diseases, ischemic renal disturbances, ischemic gastro-intestinal ulcers, has recently increased with the development of highly civilized society, and of the society holding high rates of persons of advanced age, and these diseases have become major factors in mortality rate in advanced countries.

Recently, it has been revealed that biologically activated oxygen species or reactive organic radical species play an important role in aggravation of lesions in ischemic tissues (i.e. lowering of cell function, disturbances, destruction, necrosis of cells, etc.) (I. Fridovich, Annual Review of Pharmacology and Toxicology *23,* 239 (1983); J. M. McCord, The New England Journal of Medicine, *312,* 159 (1985); K. P. Burton, J. M. McCord and G. Ghai, American Journal of Physiology, *246,* H776 (1984)]. As the active oxygen species or reactive organic radical species in living system are considered, among others, superoxide anion radical (O($O_2^-$), hydroxyl radical (.OH), singlet oxygen ($^1O_2$), and peroxide radical (ROO.). Especially, relationship between the formation of $O_2^-$ in a living system and the subsequent damages of cells or tissues caused by the reactive oxygen species have important meanings in ischemic disorders. Especially, it is considered that excess generation of ($O_2^-$ in developing tissue damages after blood reperfusion at the site of ischemic lesion or after ischemia has an important significance.

It has been known that superoxide dismutase effectively acts to scavenge ($O_2^-$ specifically, protects against tissue damages and alleviates tissue disturbances after reperfusion of the site of ischemia or after ischemia [D. N. Granger, G. Rulili, J. M. McCord, Gastroenterology, *81,* 22 (1981)]. Also, it has been reported that such compounds as ascorbic acid, α-tocopherol, cysteine and reduced glutathione have an activity to scavenge free radicals, and that these compounds could prevent lessions in tissues, which are seemed to be caused by free radicals in pathological conditions [I. Fridovich, Science, *201,* 875 (1978)], Chemistry letters, 1983, pp 631—632 and Journal of Organic Chemistry, Vol. 50, 1985, pp 281—283 describe the role of vitamin C as an antioxidant and the inhibitory efficiencies of water-soluble ascorbate and liquid-soluble ascorbyl palmitate in the autoxidation of micelle-solubilized lineolic acid. DE—A—27 29 903 discloses an ascorbic acid derivative which is effective against thrombosis].

Chemical Abstracts 75:130075n, Chemical Abstracts 73:99173a, DE—A 14 68 406, De—A 15 18 988, Chemical Abstracts 69:66921g, EP—A 0 074 411 and Chemical Abstracts 103:27082k disclose therapeutic ascorbic acid derivatives, the vitamin C activity of which corresponds to a medical indication for scurvy.

Chemical Abstracts 83:179514j describes a therapeutic ascorbic acid derivative in which the substituent at the 2-O-position is a (2-p-chlorophenoxy)-2-methylpropionyl group. EP—A—0146121 (State of the Art persuant to Ant. 54(3) EPC) discloses inter alia, 2-O-alkylascorbic acid derivatives, but does not mention their use in any method covered by Ant. 52(4) EPC.

Based on the fundamental studies so far made, revealing that reactive oxygen species and organic radicals play a significantly important role in causing tissue disturbances in a living system, the present inventors have conducted research work for finding out a novel type of pharmaceuticals aimed at scavenging reactive oxygen species and organic radicals. As the result, the present inventors found that 2-O-alkyl-substituted ascorbic acid derivatives showed, in experiments in vitro and in various animal test models, strong actions to scavenge reactive oxygen species and organic radicals, and that they controlled ischemic heart diseases, disturbances in cerebral function or renal disorders, thus accomplishing the present invention.

The present invention relates to a pharmaceutical composition for prophylaxis and improvement of disorders in functions of the circulatory system, which contains an effective amount of an ascorbic acid derivatives of the formula;

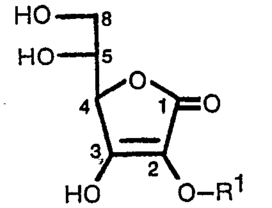

(I)

wherein $R^1$ is straight-chain alkyl having 9 to 20 carbon atoms, or a salt thereof, and a pharmaceutically acceptable carrier, vehicle or diluent therefor.

The ascorbic acid derivatives of formula [I] can be prepared by subjecting a compound representable by the formula;

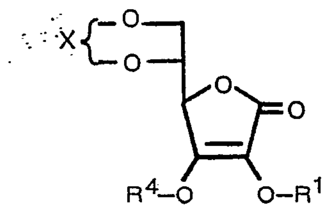

(III)

wherein $R^1$ is as defined above; $R^4$ stands for a protecting group cleavable by hydrolysis or reduction; and X stands for two hydrogens, an acetal residue or ketal residue to hydrolysis or acid hydrolysis, followed by reduction.

The above straight-chain alkyl groups have preferably 14 to 20 carbon atoms. Examples of such alkyl groups include n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosyl.

In case where Compound [I] forms a salt, the salt is exemplified by inorganic salts of such as the sodium salt, potassium salt or ammonium salt, when the protecting group in Compound [III] is cleavable by hydrolysis, can be produced by subjecting Compound [III] to acid hydrolysis to remove the acetal residue or ketal residue at the 5-, and 6-positions and the protecting group at the 3-position simultaneously.

In case where the protecting group $R^4$ at the 3-position of Compound [III] is cleavable by reduction Compound [I] can be produced by subjecting Compound [III] to acid hydrolysis to remove the acetal residuė or ketal residue at the 5,6-position, followed by removing the protecting group at the 3-position by catalytic reduction.

The above acid hydrolysis is carried out for about 1—2 hours at about 10—18°C in water or an organic solvent e.g. methanol, ethanol, dioxane, tetrahydrofuran, 1,2-dimethoxyethane, or an aqueous mixture thereof.

The above catalytic reduction is carried out for about 4—10 hours at about 10°C—100°C in an organic solvent e.g. methanol, ethanol, ethyl acetate, dioxane, 1,2-dimethoxyethane, etc., in the presence of, for example, palladium, palladium-carbon, platinum black, palladium chloride, platinum oxide, etc.

Thus produced ascorbic acid derivatives [I] can be separated and collected by *per se* known separation and purification means (e.g. column chromatography using silica-gel, polystyrene resins, activated carbon, reverse phase system, etc., recrystallization, etc.).

Compounds employable as the starting materials in the method of this invention can be produced by, for example, the following reaction steps.

[A] — Method for producing Compound [III'], i.e. Compound [III] wherein X is an acetal or ketal residue.

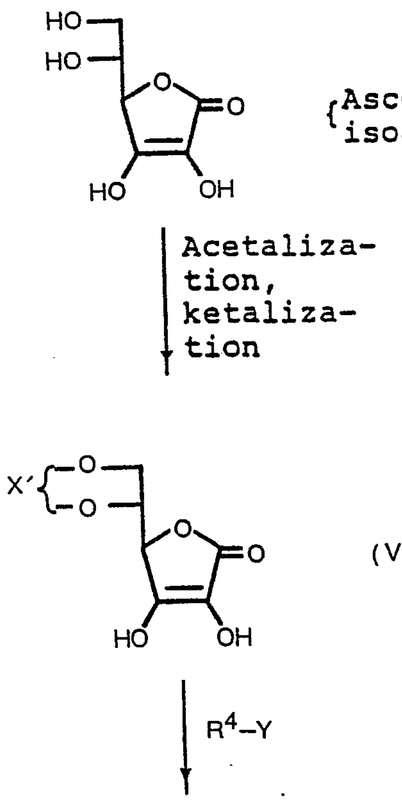


{Ascorbic acid, d,ℓ-ascorbic acid, isoascorbic acid

(VII)

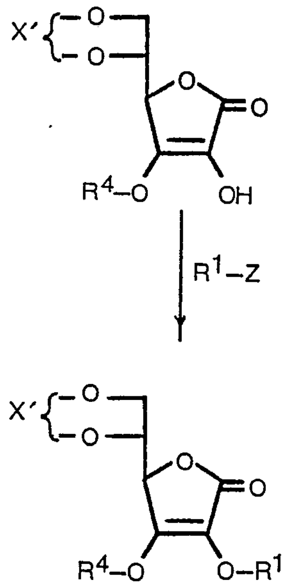


(VIII)

(III′)

In the above formulae, X′ stands for an acetal or ketal residue, $R^1$ and $R^4$ have the meaning as given hereinbefore.

In case of ascorbic acid being used as the starting material, the ascorbic acid is first acetalized or ketalized to produce Compound [VII]. This reaction is conducted by allowing ascorbic acid to react with a ketone or aldehyde e.g. acetone, benzaldehyde, cyclopentanone, cyclohexanone, etc. As the reaction solvent are employed, among others, tetrahydrofuran, chloroform, diethylether, dichloromethane or dichloroethane. The reaction temperature ranges from room temperatures to 60°C, and the reaction is conducted in the presence of an acid catalyst. Examples of the catalyst include acetylchloride, sulfuric acid, p-toluenesulfonic acid and camphorsulfonic acid. The reaction time ranges from 4 to 24 hours. Subsequently, Compound [VII] is allowed to react with a compound representable by the formula; $R^4$—Y wherein $R^4$ is as defined above, and Y stands for halogen (e.g. chlorine, bromine), (e.g. chloromethylmethylether, chloromethylethylether, benzylchloride, benzylbromide) in dimethylformamide, dimethylsulfoxide (DMSO), hexamethylphosphoramide or tetrahydrofuran, either singly or a solvent mixture thereof in the presence of an inorganic base such as potassium carbonate, sodium carbonate, sodium hydroxide, potassium hydroxide and sodium hydrogen-carbonate to produce Compound [VIII]. The reaction temperature ranges from 0°C to 40°C (preferably 25°C). The reaction goes to conclusion within 1 to 18 hours.

Then, Compound [VIII] thus obtained is allowed to react with a compound representable by the formula; $R^1$—Z wherein $R^1$ is as defined hereinbefore, and Z stands for halogen (e.g. chlorine, bromine)] in a solvent such as dimethylformamide, dimethylsulfoxide, hexamethyl-phosphoramide or tetrahydrofuran, solely or as a solvent mixture thereof, in the presence of an inorganic base (e.g. sodium hydroxide, potassium hydroxide, sodium carbonate and potassium carbonate) at a temperature of 10 to 60°C for 1 to 18 hours to produce Compound [III′].

Compound [III″], i.e. Compound [III] wherein X stands for two hydrogen atoms, can be produced by subjecting Compound [III′] to hydrolysis similar to that mentioned hereinbefore.

[B]-Compound [III″], i.e. Compound [III] wherein X is two hydrogen atoms, can be produced by the following process.

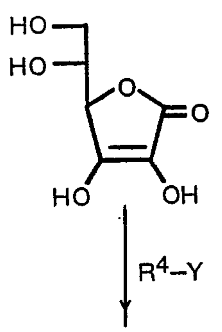


{Ascorbic acid
Isoascorbic acid

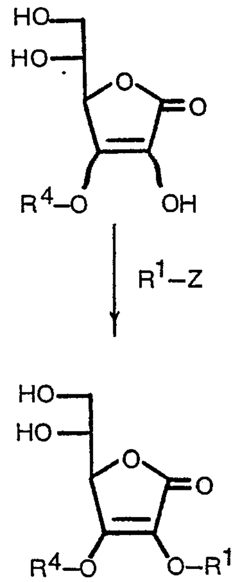


(IX)

(III''), (X)

In the above method, ascorbic acid or isoascorbic acid is employed as the starting material, and the hydroxyl group of its 3-position is allowed to react, in accordance with the conventional method, with methoxymethyl chloride, ethoxymethyl chloride, benzylbromide, trimethylsilyl chloride, dimethyl tertiary silyl chloride, etc. to give a 3-O-ether compound [IX], then, thus obtained compound [IX] is allowed to react with a compound representable by the formula; $R^1$—Z wherein $R^1$ and Z are as defined hereinbefore within the temperature range of about 10~60°C for about 1 to 20 hours in dimethylformamide, dimethylsulfoxide, hexamethyl phosphoramide, tetrahydrofuran, dioxane, etc., either singly or as a solvent mixture, in the presence of an inorganic base (e.g. potassium carbonate, sodium carbonate, etc.) to give Compound [X]. Compound [III] as produced in accordance with the afore-mentioned methods is useful as an intermediate for the synthesis of, for example, Compound [I].

Compound [I] and salts thereof show lipid per-oxidation inhibitory action in the experiments *in vitro* employing a stable radical or brain homogenates, and, in the ischemia-reperfusion model in the heart of rats or the ischemic brain model in rats or the renal failure model in rats due to oxygen free radicals, they show actions of preventing or improving the respective functional disorders, while they show remarkably low toxicity and no side effects. Compound [I] and salts thereof show therapeutic, prophylactic and improving actions against various functional disorders, for example, ischemic heart diseases (arrhythmia, coronary vasospasm, necrosis of cardiac tissue, myocardial infarction, etc.), subarachnoidal hemorrage, ischemic disorders of cerebral tissue (e.g. cerebral infarction, dementia, senile dementia, etc.), ischemic renal disorders, intestinal ischemic disorders (e.g. intestinal ulcer, etc.), thus being useful as preventing and improving agents of functional disorders in the circulatory system. Specific examples of the use as the above preventing and improving agents of functional disorders in the circulatory system include improving agents of circulatory system, improving agents of renal functions, therapeutic agents of stress intestinal ulcer, etc., e.g. agents of anti-arrhythmia, anti-myocardiac infarction, anti-cerebral infarction, preventing senile dementia, therapy and improvement after subarachmoidal hemorrhage. The compounds of this invention are low in toxicity (e.g. in acute toxicity to mice, no test animals were killed by oral administration at a dose of 1000 mg/kg), and Compound [I] can be safely administered orally or non-orally as pharmaceutical compositions [e.g. tablets, capsules (including soft-capsules and micro-capsules), liquids, suppositories, injections, preparations for nasal inhalation] prepared by mixing with *per se* conventional pharmacologically acceptable carriers, excipients, diluents, etc. in accordance with *per se* known methods. While the dosage varies with the subjects administration routes, symptoms, etc., it is usually when administered to the above-mentioned mammals, about 0.1 mg/kg~50 mg/kg body weight, preferably about 0.5 mg/kg~20 mg/kg body weight 1~3 times a day.

When Compound [I] is administered non-orally, for examples as a suppository, about 5 mg~10 mg/kg in terms of Compound [I], 1~2 times a day. As the injections, it is desirable to use, in terms of Compound [I], about 0.1 mg/kg~5 mg/kg 1~2 times a day.

For preparation of the above-mentioned compositions for oral use, a binding agent (e.g. hydroxypropyl cellulose, hydroxymethylpropylmethylcellulose, macrogol, etc.), a disintegrator (e.g. starch, carboxymethyl cellulose calcium, etc.), an excipient (e.g. lactose, starch, etc.), a lubricant (e.g. magnesium stearate, tale, etc.), etc. may be suitably incorporated.

When a composition of non-oral use, for example, a injectable preparation, an isotonizing agent (e.g. glucose, D-sorbitol, D-mannitol, sodium chloride, etc.), an antiseptic (e.g. benzylalcohol, chlorobulanol, methyl para-hydroxybenzoate, propyl para-hydroxybenzoate, etc.), a buffer (e.g. phosphate buffer, sodium acetate buffer, etc.), etc. may be suitably incorporated.

Brief Description of the Drawing

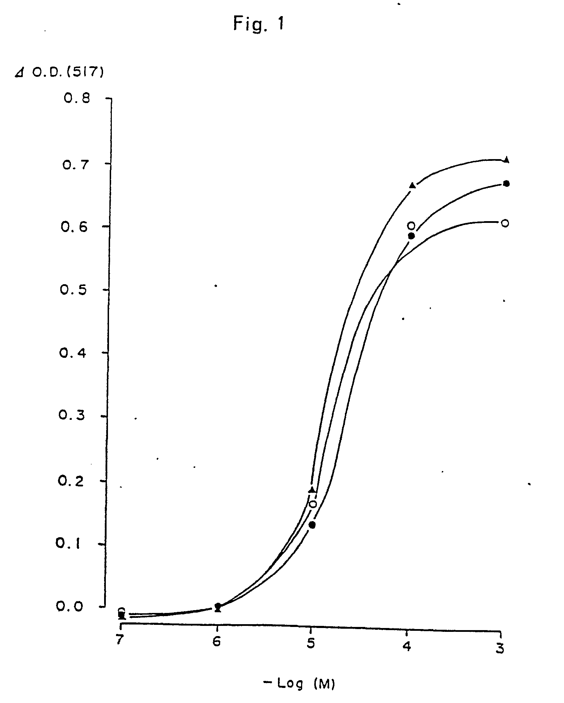

Figure 1 shows the result of oxidation inhibitory activity disclosed in Experiment 1.

The experiment examples, reference examples and working examples are given below to illustrate the present invention more specifically.

Experiment 1

Oxidation inhibitory activity determined with the use of a stable radical:

In accordance with the method of M. S. Brois [Nature, *181,* 1199, 1958], the activity to reduce a stable free radical, α, α-diphenyl-β-picryl hydrazyl (DPPH), was determined, which was used as an index of the oxidation inhibitory activity. More concretely, a test drug [i.e., the compound [I] wherein $R^1$=$-(CH_2)_{17}CH_3$, which is sometimes referred to as "compound (1—12)"] was added to 3 ml of 0.1 mM DDPH ethanol solution. Then, 20 minutes later, the absorbance at a wavelength of 517 nm was measured with the use of a spectrophotometer. The difference in absorbance between a sample solution and reference solvent [not more than 0.5% of DMF] was taken as the reduction activity.

The results of the experiment are shown in Fig. 1, where — ● — indicates the results with the above test drug, — ○ — the results with vitamin E, and — ▲ — the results with vitamin C.

The above test drug was found to reduce DDPH at the concentration of not less than $10^{-5}$M in a manner dependent upon the amount used. Vitamins C and E showed the activity equal to that of the test drug.

Experiment 2

Activity to inhibit lipid peroxide formation in rat brain tissue homogenate:

(i) Method:

Male SD rats (12-weeks old) were subjected to exsanguination under anesthesia with pentabarbitol, then the brain was excised. The brain tissue was homogenized in a phosphate buffer solution (pH 7.4) to prepare a 5% homogenate. After incubation of the homogenate at 37°C for 1 hour, the amount of lipid peroxides formed therein was determined by thiobarbituric acid (TBA) method in accordance with the report of Ohkawa et al. on Analytical Biochemistry *95* 351, 1979.

The test drug was added to the 5% homogenate before incubation to as to make the final concentration be $10^{-5}$M. The activity to inhibit the formation of lipid peroxides was compared with that of the reference group to which was added the solvent (DMSO), and shown by % inhibition.

(ii) The results are shown in Table 1 below:

The activity to inhibit the formation lipid peroxides varies with the length of the side chain, when the number (n) of the side-chain methylene groups in the general formula [I] was changed in the range of 7—21. When n is in the range of 13—19, the corresponding compounds showed higher activity, reaching 80% or higher in the inhibitory activity, which is more potent than that of vitamin E. As compared with the compound having at the 3-position a methylene chain whose n equals to 17, the compound (1—12) showed higher activity. In the same experimental system, vitamin C rather promoted the formation of lipid peroxides remarkably.

TABLE 1

Activities to inhibit lipid peroxide formation in rat brain tissue homogenates (TBA method)

| Compound [I] | Inhibitory Effect (%) |
|---|---|
| $R^2 = OH, R^3 = H, R^1 = -(CH_2)_7CH_3$ | −6.6 ± 3.0 comparative |
| $R^2 = OH, R^3 = H, R^1 = -(CH_2)_9 CH_3$ | 40.0 ± 1.1 |
| $R^2 = OH, R^3 = H, R^1 = -(CH_2)_{11}CH_3$ | 55.7 ± 26.5 |
| $R^2 = OH, R^3 = H, R^1 = -(CH_2)_{13}CH_3$ | 93.1 ± 5.3 |
| $R^2 = OH, R^3 = H, R^1 = -(CH_2)_{14}CH_3$ | 100.0 ± 0 |
| $R^2 = OH, R^3 = H, R^1 = -(CH_2)_{15}CH_3$ | 78.5 ± 11.7 |
| $R^2 = OH, R^3 = H, R^1 = -(CH_2)_{17}CH_3$ | 88.6 ± 6.8 |
| $R^2 = OH, R^3 = H, R^1 = -(CH_2)_{19}CH_3$ | 95.4 ± 4.6 |
| $R^2 = OH, R^3 = H, R^1 = -(CH_2)_{21}CH_3$ | 38.1 ± 16.6 comparative |
| Compound having a group at the 3-position* | 45.4 ± 8.7 |
| Vitamin C | −71.6 ± 36.8 |
| Vitamin E | 44.9 ± 11.7 |

Note)
The concentration of each compound is $10^{-5}$M, and the number of experimental examples of each compound is 3. Inhibitory effect (%) are shown by mean values ± standard error.

*Compound having a group at the 3-position:

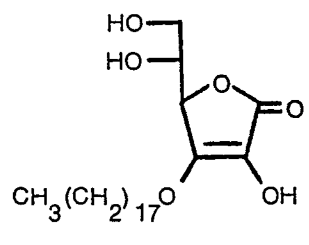

Experiment 3

Effects to alleviate renal disorders in rats due to $Fe^{3+}$ nitrilotriacetate:

(i) Method

Male SLC-Wistar rats (4 weeks old, 64 ~ 85 g) were used. The animals were housed individually in metabolism cages and allowed free access to feed and water. Body weight, volume of urine and protein in urine (BIO—RAD method) were determined every day, and occult blood reaction was also examined (Labstick method). On the last day of the experiment, the kidney was excised and weighed.

The animals were orally administered once daily with test drugs or vehicles thereof (suspension in gum arabic), and 40 ~ 60 minutes later, were injected intraperitoneally with nitrilotriacetate (NTA) or $Fe^{3+}$—NTA. $Fe^{3+}$—NTA was used in a form of mixture (1:4, molar ratio), and the dosages were 5 mg/kg in terms of $Fe^{3+}$ for 3 days and successively 10 mg/kg for 5 days.

Test drugs were compound (1—12), vitamin C and vitamin E, and the dosages were all 30 mg/kg.

(ii) Results

The results observed on the last day of the experiment are shown in the following Tablets 2 and 3.

In the animal groups administered with the vehicle, renal disorders due to $Fe^{3+}$—NTA were observed, and the weight of kidney remarkably increased and, in most of the animals, occult blood test was positive, and remarkable increases in urine volume and protein in urine were observed. In the groups administered with compound (1—12), renal disorders were alleviated; thus, as compared with the groups administered with vehicle, the weight of kidney was significantly less, and the urine volume and urinary protein were significantly less and, only in half of the test animals, occult blood was detected. Vitamin E showed substantially the same effects, but, in more than half of the test animals, occult blood test was positive. Vitamin C did not show significant effect for alleviating renal disorders.

TABLE 2

Effects on renal disorders due to $Fe^{3+}$-nitrilotriacetate (NTA)

| Group | n | Body Weight (g) | Kidney Weight (g) | Occult Blood Test |
|---|---|---|---|---|
| None*[1] | 2 | 118 | 51 | 0/2 |
| Vehicle*[2] | 6 | 86.6 ± 3.7 | 68.2 ± 2.1 | 5/6 |
| Compound (1—12) | 6 | 95.5 ± 3.0 | 56.5 ± 2.0** | 3/6 |
| Vitamin C | 6 | 89.2 ± 4.1 | 64.5 ± 1.7 | 5/6 |
| Vitamin E | 6 | 99.3 ± 2.3 | 54.3 ± 2.7** | 4/6 |

Dosage of each test drug was 30 mg/kg, orally.
n: number of test animals.
*[1] None: Control animals not administered with $Fe^{3+}$—NTA.
*[2] Vehicle: Gum arabic suspension.

TABLE 3

Effects on renal disorders due to $Fe^{3+}$-nitrilotriacetate (NTA)

| Group | n | Volume of urine (ml/day) | Protein in urine (mg/day) |
|---|---|---|---|
| None | 6 | 5.4 ± 1.2 | 3.0 ± 1.1 |
| Vehicle | 6 | 13.2 ± 2.0 | 15.1 ± 1.9 |
| Compound (1—12) | 6 | 7.4 ± 1.3* | 8.1 ± 2.1* |
| Vitamin C | 6 | 10.3 ± 1.7 | 10.7 ± 2.4 |
| Vitamin E | 6 | 9.0 ± 1.0 | 6.3 ± 1.4* |

Dosage of each test drug was 30 mg/kg, orally.
n: number of test animals.
None: Control animals not administered with $Fe^{3+}$—NTA.
Vehicle: Gum arabic suspension.

Experiment 4

Action to inhibit ventricular arrhythmias occurred during coronary artery occlusion-reperfusion in rat hearts.

(i) Method

Male SD rats (9 ~ 13-weeks old, 250 ~ 370 g) were used. The animals were subjected to thoractonomy

under artificial respiration while anesthesia was maintained by administering pentobarbital. The left anterior descending coronary artery was ligated with silk thread for 5 minutes, then the ligation was released to allow reperfusion, and animals were observed for 10 minutes. By recording standard limb lead II electrocardiograms, occurrence of ventricular arrhythmias was examined.

The animals were administered, under non-anesthesia, with test drugs as a gum arabic suspension at the dosage of 30 mg/kg at the time of about 90 minutes and of 20 mg/kg at the time of about 45 minutes (total: 50 mg/kg), or at the dosage of 10 mg/kg each (total: 20 mg/kg) at the time of about 90 minutes and about 45 minutes prior to closure of the coronary artery. The results are shown in Table 4 in terms of the total amount of the dosage.

(ii) Results

When reperfusion was permitted after the closure for 5 minutes of the left anterior descending coronary artery, ventricular arrhythmias, typically exemplified by occasionally occurring premature ventricular contractions (PVCs), ventricular tachycardia (VT) and ventricular fibrillation (VF), were observed. VT and VF were paroxysmally repeated, or sustained VF resulted in death.

In the group administered with the vehicle, VF and VT were observed in more than 90% of the animals, and the durations were respectively about 80 and 20 ~ 30 seconds. Among the animals, 10 ~ 25% were killed by occurrence of sustained VF.

In the groups administered with 20 and 50 mg/kg each of compound (1—12), occurrence of those arrhythmias was suppressed significantly and depending on the dosages. Even when the arrhythmias occurred, the period of time during which the sympton lasted was shortened. Consequently the mortality due to VF was low. Frequency of occassional PVCs was around 10 times/minutes in the groups of the vehicle, while, in the group administered with compound (1—12), the frequency was less significantly. On the other hand, no significant effects were observed by oral administration of vitamin C or E at the dosage of 50 mg/kg.

TABLE 4

Effects of ventricular arrhythmias observed when reperfusion was permitted after closure of the coronary artery in rat hearts.

| Group | Ventricular fibrillation Incidence | Ventricular fibrillation Duration | Ventricular tachycardia Incidence | Ventricular tachycardia Duration | Extrasystole | Mortality |
|---|---|---|---|---|---|---|
| Control | 7/8 (88) | 83.9 ± 27.5 | 7/8 (88) | 31.8 ± 15.0 | 10.8 ± 3.5 | 2/8 (25) |
| Compound (1—12) 50 mg/kg | 2/9* (22) | 1.2 ± 0.8** | 2/9* (22) | 3.2 ± 2.6* | 1.1 ± 0.3** | 0/9 (0) |
| Control | 16/18 (89) | 74.2 ± 30.8 | 17/18 (94) | 26.5 ± 6.8 | 11.8 ± 4.0 | 2/18 (11) |
| Compound (1—12) 20 mg/kg | 9/17* (53) | 31.0 ± 28.2 | 11/17 (65) | 10.2 ± 3.2* | 3.3 ± 0.8** | 1/17 (6) |
| Control | 17/18 (94) | 74.1 ± 36.0 | 17/18 (94) | 16.6 ± 4.4 | 7.3 ± 1.7 | 3/18 (17) |
| Vitamin C 50 mg/kg | 6/6 (100) | 9.7 ± 2.5 | 6/6 (100) | 19.0 ± 3.0 | 5.1 ± 1.0 | 0/6 (0) |
| Vitamin E 50 mg/kg | 6/10 (60) | 43.4 ± 36.0 | 7/10 (70) | 22.3 ± 9.7 | 7.0 ± 4.1 | 1/10 (10) |

*: $P < 0.05$, **: $P < 0.01$, as compared with Control Group

Incidences of ventricular fibrillation and ventricular tachycardia are shown by the percentage of the number of animals presenting the symptoms relative to the number of animals subjected to the test, and the duration of the symptoms was shown in average ±SEM by seconds.

Extrasystole is shown by the number of systole/min, and the mortality is shown by the percentage of the number of animals killed relative to the number of animals subjected to the test.

Experiment 5

Inhibition of ischemic seizure due to ligation of bilateral common carotid arteries in SHR rats.

i) Method

Male SHR rats (22-weeks old, about 360 g) were used. Under light anesthesia with ether, the animals were subjected to midline incision at the neck and the bilateral common carotid arteries were singled, then ligated to cause cerebral ischemia. Thereafter, the animals were allowed to awake from the anesthesia, and the behavior was observed for about 4 hours. The animals were orally administered with test drugs as a gum arabic suspension 60 minutes before the ligation of the bilateral common carotid arteries. The results are shown in Table 5.

ii) Results

When the bilateral common carotid arteries were ligated to cause cerebral ischemia, convulsion, an ischemic seizure, was observed after about 150 minutes in the vehicle group. The seizure was observed in about 90% of the rats within 180 minutes. But, in the group orally administered with compound (1—12) at a dosage of 100 mg/kg, the appearance of the convulsion was significantly delayed by about 40 minutes. The incidence of appearance of the seizure within 180 minutes was significantly inhibited to 20%.

TABLE 5

Activity to inhibit ischemic convulsive seizure observed when bilateral common carotid arteries were ligated in SHR rats

| | | Ischemic convulsive seizure | | |
|---|---|---|---|---|
| Group | n | Time | (min) | Incidence ⓐ |
| Vehicle | 41 | 151 | 4 | 36/41 (87.8%) |
| Compound (1—12) | 5 | 199 | 13* | 1/5* (20%) |

Compound (1—12): 100 mg/kg p.o.; Vehicle: Gum arabic suspension
ⓐ: Within 180 minutes
*: $P < 0.05$

Experiment 6

Acute toxicity in mice

(i) Method

Male Crj-ICR mice (4-weeks old, 21 ~ 26 g) were used. The animals, divided into groups, each consisting of six mice, were administered orally with compound (1—12) at the dosage of 300 or 1000 mg/kg. Then, each group was housed in a cage, and observed for 24 hours. The test drugs were suspended in gum arabic, and administered at the volume of 0.1 ml/10 g.

(ii) Results

In both groups administered with compound (1—12) at the dosages of 300 to 1000 mg/kg, the state of sedation and ptosis were observed, but both were recovered within 3 hours. During 24-hour-observation, no test animals of either group were killed.

Reference Example 1

(1) L-Ascorbic acid acetonide (42 g, 0.19 mole) was dissolved in a solvent mixture of dimethylformamide (100 ml) and hexamethylphosphoramide (100 ml), and potassium carbonate (32 g, 0.23 mole) was added to the solution, followed by ice-cooling. A solution of chloromethyl methyl ether (18 g, 0.22 mole) in tetrahydrofuran (25 ml) was added dropwise to the mixture over the 20 minutes period. After stirring at room temperature for 2.5 hours, water (200 ml) was added to the reaction mixture, to which 2*N* hydrochloric acid was added to adjust to pH 5.0, followed by extraction with four portions of ethyl acetate. The organic layer was washed with water, dried and then concentrated under reduced pressure, and the residue was subjected to silica gel column chromatography, followed by elution with isopropyl ether- ethyl acetate (2:1). The eluate was concentrated, and the residue was recrystallized from the same solvent

system to give L—5, 6-O,O-isopropylidene-3-O-methoxymethylascorbic acid (46 g). m.p. 93—94°C.

Elemental analysis, for $C_{11}H_{16}O_7$

Found: C, 50.84; H, 6.05%

Calcd.: C, 50.77; H, 6.20

(2) L-5,6-O,O-Isopropylidene-3-O-methoxymethylascorbic acid (1.84 g, 7.1 mmole) was dissolved in dimethylsulfoxide (10 ml), and octadecyl iodide (2.68 g) and potassium carbonate (1.0 g) were added to the solution, followed by allowing the reaction to proceed at 60°C for 6 hours. After the conclusion of the reaction, water (50 ml) was added to the reaction mixture, followed by extraction of the reaction product with ethyl acetate. The organic layer was washed with water, dried and concentrated under reduced pressure, and the residue was subjected to silica gel column chromatography, followed by elution with isopropyl ether. The eluate was concentrated, and the residue was recrystallized from isopropyl ether-ethyl acetate to give L-5,6-O,O-isopropylidene-3-O-methoxymethyl-2-O-octadecylascrobic acid (R1—11) (0.8 g). The physico-chemical properties are shown in Table 6.

By the same procedure as in Reference Example 1, compounds shown in Table 6 [(R1—3) to (R1—12)] were prepared.

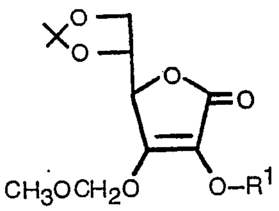

(5- and 6-positions form a isopropylidene)

TABLE 6

| Compound | $R^1$ m.p. (°C) | NMR |
|---|---|---|
| R1—3 | $-(CH_2)_8Me$ Oil | 0.85 (3H, m), 1.27 (14H, m), 1.33 (3H, s), 1.36 (3H, s), 3.48 (3H, s), 4.12 (5H, m), 4.57 (1H, d, 2Hz), 5.45 (2H, s) |
| R1—4 | $-(CH_2)_{10}Me$ Oil | 0.87 (3H, m), 1.26 (18H, m), 1.33 (3H, s), 1.37 (3H, s), 3.48 (3H, s), 4.12 (5H, m), 4.56 (1H, d, 2Hz), 5.46 (2H, s) |
| R1—5 | $-(CH_2)_{11}Me$ Oil | 0.85 (3H, m), 1.25 (20H, m), 1.34 (3H, s), 1.37 (3H, s), 3.47 (3H, s), 4.12 (5H, m), 4.55 (1H, d, 2Hz), 5.46 (2H, s) |
| R1—6 | $-(CH_2)_{12}Me$ Oil | 0.86 (3H, m), 1.25 (22H, m), 1.34 (3H, s), 1.37 (3H, s), 3.48 (3H, s), 4.13 (5H, m), 4.56 (1H, d, 2Hz), 5.45 (2H, s) |
| R1—7 | $-(CH_2)_{13}Me$ Oil | 0.86 (3H, m), 1.25 (24H, m), 1.33 (3H, s), 1.37 (3H, s), 3.45 (3H, s), 4.12 (5H, m), 4.56 (1H, d, 2Hz), 5.46 (2H, s) |
| R1—8 | $-(CH_2)_{14}Me$ Oil | 0.87 (3H, m), 1.24 (26H, m), 1.33 (3H, m), 1.37 (3H, s), 3.49 (3H, s), 4.43 (5H, m), 4.56 (1H, d, 2Hz), 5.44 (2H, s) |
| R1—9 | $-(CH_2)_{15}Me$ 44—45 | 0.87 (3H, m), 1.25 (28H, m), 1.34 (3H, s), 1.38 (3H, s), 3.50 (3H, s), 4.11 (5H, m), 4.56 (1H, d, 2Hz), 5.44 (2H, s) |
| R1—10 | $-(CH_2)_{18}Me$ Oil | 0.85 (3H, m), 1.26 (30H, m), 1.33 (3H, s), 1.36 (3H, s), 3.47 (3H, s), 4.11 (5H, m), 4.57 (1H, d, 2Hz), 5.47 (2H, s) |

TABLE 6 (continued)

| Compound | $R^1$ m.p. (°C) | NMR |
|---|---|---|
| R1—11 | —$(CH_2)_{17}$Me<br>50—52 | 0.85 (3H, m), 1.25 (32H, m), 1.33 (3H, s), 1.37 (3H, s), 3.50 (3H, m), 4.12 (5H, m), 4.57 (1H, d, 2Hz), 5.47 (2H, s) |
| R1—12 | —$(CH_2)_{19}$Me<br>57—58 | 0.85 (3H, m), 1.25 (36H, m), 1.33 (3H, s), 1.36 (3H, s), 3.47 (3H, s), 4.25 (5H, m), 4.57 (1H, d, 2Hz), 5.43 (3H, s) |

Reference Example 2

(1) L-ascorbic acid acetonide (21.6 g, 0.1 mole) was dissolved in dimethylformamide (120 ml), and the solution was cooled with ice. To the solution were added potassium carbonate (14 g, 0.1 ml) and successively benzyl bromide (11.2 ml), followed by stirring at room temperature for 20 hours. After the conclusion of the reaction, water (100 ml) was added to the reaction solution. To the mixture was added 2*N* hydrochloric acid to adjust to pH 5.0, followed by extraction with two portions of ethyl acetate. The organic layer was washed with water, dried (over magnesium sulfate) and then concentrated under reduced pressure. The product was subjected to silica gel column chromatography, and elution was effected with isopropyl ether-ethyl acetate (3:1). The eluate was concentrated, and then the residue was recrystallized from isopropyl ether-ethyl acetate to give L-5,6-O,O-isopropylidene-3-O-benzylascorbic acid (13 g, 40%), m.p. 105—106°C.

(2) L-5,6-O,O-isopropylidene-3-O-benzylascorbic acid (3.06 g, 0.01 mole) was dissolved in a solvent mixture of dimethyl-sulfoxide (20 ml) and tetrahydrofuran (15 ml), and potassium carbonate (1.5 g, 0.011 mole) was added to the solution. Then, octadecyl iodide (3.83 g) was added to the mixture, followed by stirring at room temperature for 18 hours. After the conclusion of the reaction, water (100 ml) was added to the reaction mixture, followed by extraction with ethyl acetate. The organic layer was washed with water, dried (over magnesium sulfate) and then concentrated under reduced pressure. The residue was subjected to silica-gel column chromatography, followed by elution with isopropyl ether-ethyl acetate (10:1) to give L-5,6-O,O-isopropylidene-3-O-benzyl-2-O-octadecyl-ascorbic acid [compound (R2—7)] (3.8 g). The physico-chemical properties are shown in Table 7.

By the same procedure as in Reference Example 2, compounds shown in Table 7 [(R 2—2) to (R2—8)] were prepared.

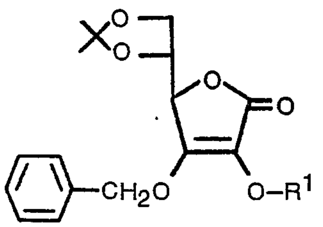

(5- and 6- positions form an isopropylidene)

TABLE 7

| Compound | $R^1$ m.p. (°C) | NMR |
|---|---|---|
| R2—2 | —$(CH_2)_9$Me<br>Oil | 0.86 (3H, m), 1.26 (16H, m), 1.34 (3H, s), 1.37 (3H, s), 4.08 (5H, m), 4.54 (1H, d, 2Hz), 5.46 (2H, s), 7.37 (5H, s) |
| R2—3 | —$(CH_2)_{13}$Me<br>Oil | 0.86 (3H, m), 1.24 (26H, m), 1.34 (3H, s), 1.37 (3H, s), 4.08 (5H, m), 4.53 (1H, d, 2Hz), 5.46 (2H, s), 7.36 (5H, s) |
| R2—4 | —$(CH_2)_{14}$Me<br>Oil | 0.86 (3H, m), 1.26 (26H, m), 1.34 (3H, s), 1.37 (3H, s), 4.10 (5H, m), 4.53 (1H, d, 2Hz), 5.46 (2H, s), 7.36 (5H, s) |

TABLE 7 (continued)

| Compound | $R^1$ m.p. (°C) | NMR |
|---|---|---|
| R2—5 | —$(CH_2)_{15}Me$ Oil | 0.85 (3H, m), 1.25 (26H, m), 1.34 (3H, s), 1.37 (3H, s), 4.06 (5H, m), 4.54 (1H, d, 2Hz), 5.46 (2H, s), 7.37 (5H, s) |
| R2—6 | —$(CH_2)_{16}Me$ Oil | 0.87 (3H, m), 1.25 (28H, m), 1.34 (3H, s), 1.37 (3H, s), 4.08 (5H, m), 4.54 (1H, d, 2Hz), 5.46 (2H, s), 7.36 (5H, s) |
| R2—7 | —$(CH_2)_{17}Me$ 44—45 | 0.88 (3H, m), 1.26 (32H, m), 1.38 (6H, s), 4.08 (5H, m), 4.51 (1H, d, 2Hz), 5.43 (2H, s), 7.29 (5H, s) |
| R2—8 | —$(CH_2)_{19}Me$ 48—50 | 0.87 (3H, m), 1.24 (36H, m), 1.34 (3H, s), 1.37 (3H, s), 4.06 (5H, m), 4.54 (1H, d, 2Hz), 5.46 (2H, s), 7.36 (5H, s) |

Reference Example 3

(1) L-5,6-O,O-Isopropylidene-3-O-benzyl-2-O-octadecylascorbic acid (3.8 g) was dissolved in a solvent mixture of tetrahydrofuran (40 ml) and methanol (10 ml), and 2*N* hydrochloric acid (20 ml) was added to the solution, followed by stirring at 50°C for 24 hours. After the conclusion of the reaction, the reaction solution was concentrated under reduced pressure, and the reaction product was extracted with ethyl acetate. The organic layer was washed with water, dried and then concentrated under reduced pressure, and the residue was recrystallized from isopropyl ether-ethyl acetate to give 3-O-benzyl-2-O-octadecylascorbic acid [compound (R3—7)] (2.6 g).

The physico-chemical properties are shown in Table 8.

(2) The compounds obtained in Reference Example 2 were processed by a procedure analogous to the above to prepare compounds [(R3—2) to (R3—8)], which are shown in Table 8.

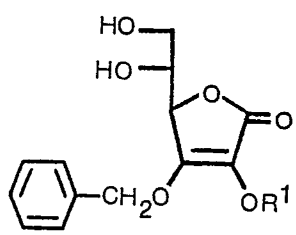

TABLE 8

| Compound | $R^1$ m.p. (°C) | NMR |
|---|---|---|
| R3—2 | —$(CH_2)_9Me$ Oil | 0.86 (3H, m), 1.25 (16H, m), 3.80 (2H, m), 4.00 (3H, m), 4.68 (1H, d, 2Hz), 5.46 (2H, s), 7.36 (5H, s) |
| R3—3 | —$(CH_2)_{13}Me$ 65—66 | 0.86 (3H, m), 1.25 (24H, m), 3.80 (2H, m), 4.00 (3H, m), 4.68 (1H, d, 2Hz), 5.47 (2H, s), 7.35 (5H, s) |
| R3—4 | —$(CH_2)_{14}Me$ 62—63 | 0.87 (3H, m), 1.23 (26H, m), 3.82 (2H, m), 4.02 (3H, 4.68 (1H, d, 2Hz), 5.47 (2H, s), 7.36 (5H, s) |
| R3—5 | —$(CH_2)_{15}Me$ 71—72 | 0.86 (3H, m), 1.25 (28H, m), 3.85 (2H, m), 4.02 (3H, m), 4.69 (1H, d, 2Hz), 5.47 (2H, s), 7.37 (5H, s) |

TABLE 8 (continued)

| Compound | $R^1$ m.p. (°C) | NMR |
|---|---|---|
| R3—6 | —$(CH_2)_{16}$Me 57—58 | 0.88 (3H, m), 1.25 (30H, m), 3.79 (2H, m), 4.00 (3H, m), 4.67 (1H, d, 2Hz), 5.44 (2H, s), 7.33 (5H, s) |
| R3—7 | —$(CH_2)_{17}$Me 75—76 | 0.87 (3H, m), 1.25 (32H, m), 3.80 (2H, m), 4.01 (3H, m), 4.68 (1H, m), 5.46 (2H, s), 7.34 (5H, s) |
| R3—8 | —$(CH_2)_{19}$Me 77—78 | 0.86 (3H, m), 1.24 (36H, m), 4.00 (5H, m), 4.68 (1H, m), 5.46 (2H, s), 7.36 (5H, s) |

Reference Example 4

L-5,6-O,O-Isopropylidene ascorbic acid (52 g, 0.24 mole) was dissolved in a mixture of tetrahydrofuran (200 ml) and DMF (50 ml). Potassium carbonate (42 g, 0.3 mole) was added to the reaction mixture and the mixture was stirred for 10 minutes at room temperature. To the reaction mixture chloromethyl ethyl ether (27 ml, 0.3 mole) was added during 5 minutes under keeping about 20°C in an ice bath. After stirring for 4 hours at room temperature, the reaction mixture was poured onto water (300 ml), adjusted at pH 8 by adding 2*N* hydrochloric acid and then extracted twice (400 ml, 200 ml) with ethyl acetate. The organic layer was washed with water, dried and concentrated under reduced pressure. The resulting crude product was purified with silica gel column chromatography (300 g, Merck Ltd. Art 7734, developing solvent; ethyl acetate:isopropyl ether = 1:2) and then recrystallized from isopropyl ether (crystallized out in refrigerator) to give 5,6-O,O-isopropylidene-3-O-ethoxymethyl-(L)-ascorbic acid (40 g, 61%).

Reference Example 5

L-5,6-O,O-Isopropylidene-3-O-ethoxymethyl ascorbic acid (13.6 g, 0.05 mole) and octadecyl iodide (20 g, 0.055 mole) were dissolved in a mixture of tetrahydrofuran (200 ml) and DMSO (50 ml), and added potassium carbonate (8 g, 0.06 mole) to the reaction mixture under stirring at room temperature. After stirring the mixture for 3 hours at 50°C, water (300 ml) was added to the mixture. The reaction mixture was adjusted to pH 7 with 2*N* hydrochloric acid and extracted with isopropyl ether (600 ml). The organic layer was washed with water, dried and concentrated under reduced pressure. The resulting crude product was purified with silica gel column chromatography (300 g, developing solvent; isopropyl ether) to give 5,6-O,O-isopropylidene-3-O-ethoxymethy-2-O-octedecyl-(L)-ascorbic acid (15 g, 57%). This compound did not form any crystals at room temperature.

Reference Example 6

(1) To a solution of 2-O-octadecyl-L-ascorbic acid (0.8 g, 2 mmole) in cloroform (20 ml) was added pyridine (1 ml), followed by adding dropwise benzoyl chloride (0.28 g, 2 mmole) at room temperature. The reaction mixture was stirred for one hour, to which was added 2*N* hydrochloric acid to acidify it. The organic layer was washed with water and dried (over magnesium sulfate). The solvent was evaporated off, and the product was recrystallized from isopropyl ether-ethyl acetate to give 3-O-benzoyl-2-O-octadecyl-L-ascorbic acid (0.6 g, 49%), m.p. 68—69°C. $C_3H_{48}O_7$ (Found: C, 69.94; H, 8.98%. Anal. Calcd: C, 69.89; H, 9.08).

(2) By the same procedure as in the above, 2-O-hexadecyl-L-ascorbic acid was subjected to benzoylation to give 3-O-benzoyl-2-O-hexadecyl-L-ascorbic acid, m.p. 77—78°C. $C_{29}H_{44}O_7$ (Found: C, 69.21; H, 8.82%. Anal. Calcd.: C, 69.02; H, 8.79).

Example 1

L-5,6-O,O-Isopropylidene-3-O-methoxymethyl-2-O-octadecylascorbic acid (1.2 g) was dissolved in a solvent mixture of methanol (30 ml) and tetrahydrofuran (10 ml), and 2*N* hydrochloric acid (10 ml) was added to the solution, followed by stirring at 50°C for 6 hours. The reaction solution was concentrated under reduced pressure, and the reaction product was extracted with ethyl acetate. The organic layer was washed with water, dried (over magnesium sulfate) and then concentrated under reduced pressure, and the residue was recrystallized from isopropyl ether-ethyl acetate to give 2-O-octadecylascorbic acid [compound (1—12)] (0.82 g). This compound was also obtained from L-5,6-O,O-isopropylidene-3-O-ethoxymethyl ascorbic acid by hydrolysis mentioned above. The physico-chemical properties are shown in Table 9.

Example 2

3-O-Benzyl-2-O-octadecylascorbic acid (2.1 g) was dissolved in ethyl acetate (25 ml), and 5% Pd-C (0.5 g) was added to the solution to conduct catalytic reduction under atmospheric pressure. The catalyst was filtered out, and the filtrate was concentrated under reduced pressure. The reaction product was recrystallized from isopropyl ether-ethyl acetate to give 2-O-octadecylascorbic acid [compound (1—12)] (1.5 g).

The physico-chemical properties are shown in Table 9.

Example 3

Compounds [(1—3) to (1—13)] prepared by a procedure similar to the above Examples 1 and 2 are set forth in Table 9.

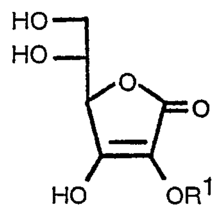

TABLE 9

| Compound | $R^1$ | Ex. No. depended | m.p. (°C) | NMR |
|---|---|---|---|---|
| 1—3 | $—(CH_2)_8Me$ | 1, 2 | 122—123 | 0.86 (3H, m), 1.25 (14H, m), 3.54 (2H, m), 3.87 (3H, m), 4.71 (1H, d, 1Hz) |
| 1—4 | $—(CH_2)_9Me$ | 2 | 118—120 | 0.85 (3H, m), 1.24 (16H, m), 3.43 (2H, m), 3.86 (3H, m), 4.72 (1H, d, 1Hz) |
| 1—5 | $—(CH_2)_{10}Me$ | 1 | 124—125 | 0.86 (3H, m), 1.24 (18H, m), 3.50 (2H, m), 3.87 (3H, m), 4.71 (1H, d, 1Hz) |
| 1—6 | $—(CH_2)_{11}Me$ | 1 | 127—128 | 0.85 (3H, m), 1.24 (20H, m), 3.53 (2H, m), 3.91 (3H, m), 4.72 (1H, d, 1Hz) |
| 1—7 | $—(CH_2)_{12}Me$ | 1 | 129—130 | 0.85 (3H, m), 1.25 (22H, m), 3.52 (2H, m), 3.91 (3H, m), 4.72 (1H, d, 1Hz) |
| 1—8 | $—(CH_2)_{13}Me$ | 1, 2 | 126—127 | 0.85 (3H, m), 1.25 (24H, m), 3.51 (2H, m), 3.90 (3H, m), 4.74 (1H, d, 1Hz) |
| 1—9 | $—(CH_2)_{14}Me$ | 1, 2 | 126—127 | 0.85 (3H, m), 1.26 (26H, m), 3.45 (2H, m), 3.86 (3H, m), 4.73 (1H, d, 1Hz) |
| 1—10 | $—(CH_2)_{15}Me$ | 1, 2 | 128—129 | 0.86 (3H, m), 1.24 (28H, m), 3.59 (2H, m), 3.94 (3H, m), 4.75 (1H, d, 1Hz) |
| 1—11 | $—(CH_2)_{16}Me$ | 1, 2 | 127—129 | 0.86 (3H, m), 1.27 (30H, m), 3.54 (2H, m), 3.86 (3H, m), 4.71 (1H, d, 1Hz) |

TABLE 9 (continued)

| Compound | $R^1$ | Ex. No. depended | m.p. (°C) | NMR |
|---|---|---|---|---|
| 1—12 | $—(CH_2)_{17}Me$ | 1, 2 | 127—128 | 0.85 (3H, m), 1.26 (32H, m), 3.51 (2H, m), 3.91 (3H, m), 4.75 (1H, d, 1Hz) |
| 1—13 | $—(CH_2)_{19}Me$ | 1, 2 | 126—127 | 0.85 (3H, m), 1.23 (36H, m), 3.45 (2H, m), 3.86 (3H, m), 4.70 (1H, d, 1Hz) |

Example 4

1) To a solution of sodium D-isoascorbate 120 g. 0.1 mole) in dimethylformamide (50 ml) was added dropwise benzyl bromide (12 ml). The mixture was heated at 50°C for 4 hours. To the reaction solution was added water (100 ml). The product was extracted with ethyl acetate. The organic layer was washed with water, dried and concentrated. Then, the crude product was subjected to silica-gel chromatography and developed with ethyl acetate to give 2-O-benzyl-D-isoascorbic acid (10 g, 37%). This benzyl compound (10 g, 0.037 mole) was dissolved in a mixture of dimethyl sulfoxide (40 ml) and tetrahydrofuran (10 ml). The solution was allowed to react with octadecyl iodide (14 g) in the presence of potassium carbonate (5 g) at 50°C for 2 hours. To the reaction product, after cooling, was added water (100 ml), and the product was extracted with isopropyl ether. The organic layer was washed with water, dried and concentrated under reduced pressure. The concentrate was subjected to silica-gel column chromatography, followed by development with isopropyl ether: ethyl acetate (1:1). Thus-obtained crude crystals were recrystallized from hexane: isopropyl ether (1:1) to give 2-O-octadecyl-3-O-benzyl-D-isoascorbic acid (5 g, 26%), m.p. 62—63°C. $C_{31}H_{50}O_8$ (Found: C, 72.02; H, 9.67%. Anal. Calcd: C, 71.78; H, 9.72).

2) 2-O-Octadecyl-3-O-benzyl-D-isoascorbic acid obtained as above (3 g, 6.7 mmole) was dissolved in ethanol (50 ml). The solution was subjected to hydrogenation under atmospheric pressure in the presence of 5% Pd-carbon (0.2 g). Eighteen hours later, the catalyst was filtered off, and the filtrate was concentrated under reduced pressure. The concentrate was recrystallized from ethyl acetate to give 2-O-octadecyl-D-isoascorbic acid (2 g, 80%), m.p. 103—104°C. $C_{24}H_{44}O_6$ (Found: C, 67.45; H, 10.46%. Anal. Calcd: C, 67.26; H, 10.35).

Example 5

By using the following ingredients, tablets are prepared by *per se* conventional means.

| | |
|---|---|
| Compound (1—12) [Compound [I] wherein $R^1=(CH_2)_{17}CH_3$ $R^2=OH$, $R^3=H$] | 50 mg |
| Corn starch | 90 mg |
| Lactose | 30 mg |
| Hydroxypropyl cellulose L | 25 mg |
| Magnesium stearate | 5 mg |
| Total | 200 mg (per tablet) |

Dosage is 1—3 tablets/adult after each meal (three times/day).

Example 6

By using the following ingredients, tablets are prepared by *per se* conventional means.

| | |
|---|---|
| Compound (1—9) | |
| [Compound [I] wherein $R^1=(CH_2)_{14}CH_3$ $R^2=OH$, $R^3=H$] | 60 mg |
| Corn starch | 80 mg |
| Lactose | 30 mg |
| Hydroxypropyl cellulose L | 25 mg |
| Magnesium stearate | 5 mg |
| Total | 200 mg (per tablet) |

Dosage is 1—3 tablets/adult after each meal (three times/day).

**Claims for Contracting States: BE CH DE FR GB IT LI LU NL SE**

1. A pharmaceutical composition for the prophylaxis or improvement of disorders in functions of the circulatory system, which contains an effective amount of an ascorbic acid derivative of the formula:

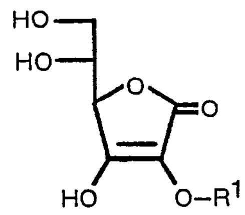

(I)

wherein $R^1$ is straight-chain alkyl having 9 to 20 carbon atoms, or a salt thereof, and a pharmaceutically acceptable carrier, vehicle or diluent therefor.

2. The composition according to claim 1, wherein the ascorbic acid derivative is selected from 2-O-pentadecylascorbic acid and 2-O-octadecylascorbic acid.

3. The composition according to claim 1, wherein the composition is formulated as a tablet.

**Claims for contacting state: AT**

1. A method for producing a pharmaceutical composition for prophylaxis or improvement of disorders in functions of the circulatory system, which contains an effective amount of an ascorbic acid derivative of the formula:

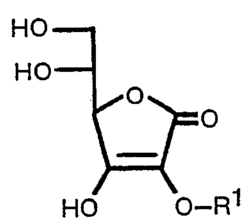

(I)

wherein $R^1$ is straight-chain alkyl having 9 to 20 carbon atoms or a salt thereof, and a pharmaceutically acceptable carrier, vehicle or diluent therefor, which comprises admixing the ascorbic acid derivative with the pharmaceutically acceptable carrier, vehicle or diluent therefor.

2. The method according to claim 1, wherein the ascorbic acid derivative is selected from 2-O-pentadecylascorbic acid and 2-O-octadecylascorbic acid.

3. The method according to claim 1, wherein the composition is formulated as a tablet.

**Patentansprüche für die Vertragsstaaten: BE CH DE FR GB IT LI LU NL SE**

1. Pharmazeutische Zusammensetzung zur Prophylaxe oder Besserung von Funktionsstörungen des Kreislaufsystems, die eine wirksame Menge eines Ascorbinsäurederivates der Formel

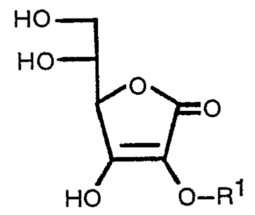

(I)

worin $R^1$ ein geradkettiges Alkyl mit 9 bis 20 Kohlenstoffatomen ist, oder ein Salz derselben, une ein pharmazeutisch verträgliches Trägermittel, Vehikel oder Verdünnungsmittel dafür enthält.

2. Zusammensetzung nach Anspruch 1, worin das Ascorbinsäurederivat aus 2-O-Pentadecylascorbinsäure und 2-O-Octadecylascorbinsäure gewählt ist.

3. Zusammensetzung nach Anspruch 1, worin die Zusammensetzung als Tablette formuliert ist.

**Patentansprüche für den Vertragsstaat: AT**

1. Verfahren zur Herstellung einer pharmazeutischen Zusammensetzung zur Prophylaxe oder Besserung von Funktionsstörungen des Kreislaufsystems, die eine wirksame Menge eines Ascorbinsäurederivates der Formel

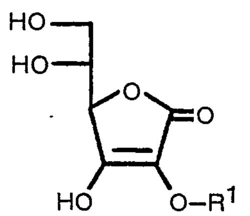

(I)

worin $R^1$ ein geradkettiges Alkyl mit 9 bis 20 Kohlenstoffatomen ist, oder ein Salz derselben, und ein pharmazeutisch verträgliches Trätermittel, Vehikel oder Verdünnungsmittel dafür enthält, umfassend das Vermischen des Ascorbinsäurederivates mit dem pharmazeutisch verträglichen Trägermittel, Vehikel oder Verdünnungsmittel dafür.

2. Verfahren nach Anspruch 1, worin das Ascorbinsäurederivat aus 2-O-Pentadecylascorninsäure und 2-O-Octadecylascorbinsäure gewählt ist.

3. Verfahren nach Anspruch 1, worin die Zusammensetzung als Tablette formuliert ist.

**Revendications pour les Etats contractants: BE CH DE FR GB IT LI LU NL SE**

1. Composition pharmaceutique pour la prophylaxie ou le traitement de toubles fonctionnels du système circulatoire, laquelle contient une quantité efficace d'un dérivé d'acide ascorbique de formule

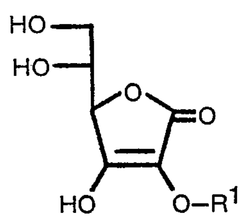

(I)

dans laquelle $R^1$ est un groupe alkyle à chaîne droite ayant de 9 à 20 atomes de carbone, ou d'un sel de celui-ci, et un véhicule, excipient ou diluant pharmaceutiquement acceptable pour ce dérivé.

2. Composition selon la revendication 1, dans laquelle le dérivé d'acide ascorbique est choisi parmi l'acide 2-O-pentadécylascorbique et l'acide 2-O-octadécylascorbique.

3. Composition selon la revendication 1, dans laquelle la composition est formulée sous forme de comprimé.

**Revendications pour l'Etat contractant: AT**

1. Procédé de production d'une composition pharmaceutique pour la prophylaxie ou le traitement de troubles fonctionnels du système circulatoire, laquelle contient une quantité efficace d'un dérivé d'acide ascorbique de formule

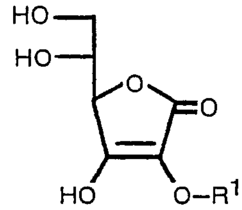

(I)

dans laquelle $R^1$ est un groupe alkyle à chaîne droite ayant de 9 à 20 atomes de carbone, ou d'un sel de celui-ci, et un véhicule, excipient ou diluant pharmaceutiquement acceptable pour ce dérivé, qui comporte le mélange du dérivé d'acide ascorbique avec le véhicule, excipient ou diluant pharmaceutiquement acceptable pour ce dérivé.

2. Procédé selon la revendication 1, dans laquelle le dérivé d'acide ascorbique est chosi parmi l'acide 2-O-pentadécylascorbique et l'acide 2-O-octadécylascorbique.

3. Procédé selon la revendication 1, dans laquelle la composition est formulée sous forme de comprimé.

Fig. 1